# EUROPEAN PATENT SPECIFICATION

(11) **EP 2 583 689 B1**
(45) Date of publication and mention of the grant of the patent: **31.08.2016**
(21) Application number: 12189251.7
(22) Date of filing: 19.10.2012
(51) Int. Cl.: A61K 41/00, A61K 47/48

(54) **Liposome including elastin-like polypeptides and use thereof**
Liposom mit elastinartigen Polypeptiden und Verwendung davon
Liposome comprenant des polypeptides de type élastine et son utilisation

(30) Priority: 19.10.2011 KR 20110107055
(43) Date of publication of application: 24.04.2013
(73) Proprietor: Samsung Electronics Co., Ltd, Gyeonggi-do 443-742 (KR)
(72) Inventor: Kim, Min-sang, Gyeonggi-do (KR); Kim, Hyun-ryoung, Gyeonggi-do (KR); Park, Sun-min, Gyeonggi-do (KR); Park, Jae-chan, Gyeonggi-do (KR); Chae, Su-young, Gyeonggi-do (KR)
(74) Representative: Grünecker Patent- und Rechtsanwälte PartG mbB

(56) References cited:
- US-A- 5 720 976
- BIKRAM M ET AL: "Thermo-responsive systems for controlled drug delivery", EXPERT OPINION ON DRUG DELIVERY 200810 GB, vol. 5, no. 10, October 2008 (2008-10), pages 1077-1091, XP8159163, ISSN: 1742-5247
- CHILKOTI A ET AL: "Design of thermally responsive, recombinant polypeptide carriers for targeted drug delivery", ADVANCED DRUG DELIVERY REVIEWS 20021018 NL, vol. 54, no. 8, 18 October 2002 (2002-10-18), pages 1093-1111, XP002690721, ISSN: 0169-409X
- WU X S ET AL: "Conjugation of phosphatidylethanolamine to poly(N-isopropylacrylamide) for potential use in liposomal drug delivery systems", POLYMER, ELSEVIER SCIENCE PUBLISHERS B.V, GB, vol. 33, no. 21, 1 January 1992 (1992-01-01), pages 4659-4662, XP024117511, ISSN: 0032-3861, DOI: 10.1016/0032-3861(92)90432-V [retrieved on 1992-01-01]
- KYUNGA NA ET AL: "Elastin-like polypeptide modified liposomes for enhancing cellular uptake into tumor cells", COLLOIDS AND SURFACES. B, BIOINTERFACES, ELSEVIER, AMSTERDAM, NL, vol. 91, 27 October 2011 (2011-10-27), pages 130-136, XP028347797, ISSN: 0927-7765, DOI: 10.1016/J.COLSURFB.2011.10.051 [retrieved on 2011-11-02]

## Description

### LIPOSOME INCLUDING ELASTIN-LIKE POLYPEPTIDES AND USE THEREOF BACKGROUND

The present disclosure relates to a liposome comprising elastin-like polypeptides (ELPs), a pharmaceutical composition comprising the liposome, and the liposomes for use in a method of delivering active agents to a target site.

Liposomes have at least one lipid bilayer membrane enclosing an aqueous internal compartment. Liposomes may be characterized by membrane type and by size. Small unilamellar vesicles (SUVs) have a single membrane and typically range between 20 and 50 nm in diameter. Large unilamellar vesicles (LUVs) are typically larger than 50 nm. Oligolamellar large vesicles and multilamellar vesicles have multiple, usually concentric, membrane layers and are typically larger than 100 nm. Liposomes with several nonconcentric membranes, i.e., several smaller vesicles contained within a larger vesicle, are termed multivesicular vesicles.

Liposomes are formulated to carry drugs or other active agents either contained within the aqueous interior space (water-soluble active agents) or partitioned into the lipid bilayer (lipid-soluble active agents).

Active agents which have short half-lives in the bloodstream are particularly suited to delivery via liposomes. Many anti-neoplastic agents, for example, are known to have a short half-life in the bloodstream and thus, their parenteral use is not feasible. However, the use of liposomes for site-specific delivery of active agents via the bloodstream is severely limited by the rapid clearance of liposomes from the blood by cells of the reticuloendothelial system (RES).

Liposomes are not normally leaky unless a hole is formed in the liposome membrane, unless the membrane degrades or dissolves, or unless a temperature of the membrane increases to a phase transition temperature. The elevation of temperature at a target site in a subject (hyperthermia) may increase the temperature of the liposome to a phase transition temperature or higher and thus liposome contents may be released. This procedure may be used for the selective delivery of therapeutic agents. However, this technique is limited where the phase transition temperature of the liposome is significantly higher than the normal tissue temperature.

It is accordingly desirable to devise liposome formulations capable of efficiently delivering active agents.

### SUMMARY

Provided is a liposome comprising elastin-like polypeptides (ELPs).

Provided is a pharmaceutical composition comprising the liposome.

Provided is a liposome for use in a method of delivering active agents to a target site in a subject using the liposome.

### EMBODIMENTS OF THE INVENTION

(1) A liposome comprising:
   a lipid bilayer;
   an elastin-like polypeptide (ELP) conjugated to a hydrophobic moiety, wherein the hydrophobic moiety is in the lipid bilayer; and
   a lipid bilayer stabilizing agent, wherein the ELP is an amino acid polymer that is soluble in an aqueous solution below an inverse transition temperature (Tₜ) and is insoluble as the temperature is raised higher than the an inverse transition temperature (Tₜ).
(2) The liposome of (1), wherein stabilizing agent comprises a steroid.
(3) The liposome of (1), wherein the stabilizing agents are one selected from the group consisting of sterols, sphingolipids, and combinations thereof.
(4) The liposome of (1), wherein the stabilizing agents are one selected from the group consisting of cholesterols, sitosterols, ergosterols, stigmasterols, 4,22-stigmastadien-3-ones, stigmasterol acetates, lanosterols, cycloartenols, and combinations thereof.
(5) The liposome of (1), wherein the ELP comprises one or more repeating units of VPGXG, PGXGV, GXGVP, XGVPG, GVPGX, or combinations thereof, wherein V is valine, P is proline, G is glycine, and X is any amino acid except proline.
(6) The liposome of (5), wherein the repeating units are repeated 2 to 200 times.
(7) The liposome of (1), wherein the hydrophobic moiety conjugated to the ELP comprises a hydrophobic molecule or an amphipathic molecule.
(8) The liposome of (1), wherein the hydrophobic moiety conjugated to the ELP comprises saturated or unsaturated hydrocarbons, saturated or unsaturated acyl molecules, or saturated or unsaturated alkoxy molecules.
(9) The liposome of (1), wherein the lipid bilayer comprises one or more phospholipids.
(10) The liposome of (9), wherein the one or more phospholipids comprise a phosphatidyl choline, phosphatidyl glycerol, phosphatidyl inositol, phosphatidyl ethanolamine, or combination thereof.
(11) The liposome of (9), wherein the one or more phospholipids includes a phospholipid comprising one or more acyl groups having 16-24 carbon atoms.
(12) The liposome of (9), wherein the one or more phospholipids include a phospholipid derivatized with a hydrophilic polymer.
(13) The liposome of (12), wherein the hydrophilic polymer is polyethylene glycol, polylactic acid, polyglycolic acid, a copolymer of polylactic acid and polyglycolic acid, polyvinyl alcohol, polyvinyl pyrrolidone, oligosaccharide, or a mixture thereof.
(14) The liposome of (1), wherein the lipid bilayer comprises phospholipids, phospholipids derivatized with hydrophilic polymers, and cholesterols.
(15) The liposome of (1), wherein the liposome has a phase transition temperature of 39 °C to 45 °C.
(16) The liposome of (1), wherein the liposome has a diameter of 50 nm to 500 nm.
(17) The liposome of (1), wherein the liposome further comprises one or more active agents.
(18) The liposome of (17), wherein the one or more active agents are contained in an interior space of the liposome, in an interior region of the lipid bilayer, on the surface of the lipid bilayer, or combination thereof.
(19) The liposome of (17), wherein the one or more active agents comprise a pharmacologically active agent, diagnostic agent, or combination thereof.
(20) The liposome of (19), wherein the one or more active agents comprise an anesthetic, antihistamine, antineoplastic, anti-ulcerative, anti-seizure agent, muscle relaxant, immunosuppressive agent, anti-infective agent, non-steroidal anti-inflammatory agent, imaging agent, nutritional agent, or a combination thereof.
(21) A pharmaceutical composition comprising:
   the liposome of (17) and,
   a pharmaceutically acceptable carrier or diluent.
(22) The liposome of (17) for use in a method of delivering one or more active agents to a target site in a subject.
(23) A method of preparing a liposome comprising combining one or more bilayer-forming lipids;
   one or more elastin-like polypeptides (ELPs) each conjugated to a hydrophobic moiety; and
   one or more lipid bilayer stabilizing agents;
   to provide a liposome.
(24) The method of (23), wherein the liposome comprises a lipid bilayer, and the hydrophobic moiety conjugated to the one or more ELPs is in the lipid bilayer.
(25) The method of (23), wherein the one or more bilayer-forming lipids comprise a phospholipid.
(26) The method of (23), further comprising combining one or more active agents with the one or more bilayer-forming lipids, one or more ELPs each conjugated to a hydrophobic moiety, and one or more lipid bilayer stabilizing agents, or combining one or more active agents with the liposome, to provide a liposome containing the one or more active agents.
(27) The method of (23), wherein the method comprises
   combining the one or more bilayer-forming lipids provided in a first solvent with the one or more ELPs each conjugated to a hydrophobic moiety and the one or more lipid stabilizing agents provided in a second solvent;
   evaporating the combined solvents to provide a lipid layer;
   hydrating the lipid layer with an aqueous solvent;
   and filtering the hydrated lipid layer to provide a liposome.
(28) The method of (27), wherein the aqueous solvent contains one or more active agents.
(29) The method of (27), comprising combining one or more active agents with the liposome in the presence of pH gradient or ammonium sulfate gradient between the inside and outside of the liposome.

### BRIEF DESCRIPTION OF THE DRAWINGS

FIG. 1 is a graph showing the temperature release profiles of calcein from the liposomes prepared in Example 1 using stearoyl-VPGVG VPGVG VPGVG VPGVG VPGVG VPGVG-NH₂ (SA-V6-NH₂), 1,2-dipalmitoyl-sn-glycero-3-phosphocholine (DPPC), [1,2-distearoyl-sn-glycero-3-phosphoethanolamine-N-[methoxy(polyethylene glycol)-2000] (ammonium salt)] (DSPE-PEG-2000) and cholesterols in a molar ratio of about 0.55:about 55:about 2:about 20 or about 0.55:about 55:about 2:about 40, where 20% and 40% of cholesterols per whole lipids were used.
FIG. 2 is a graph showing the temperature release profiles of calcein from the liposomes prepared by using SA-V6-NH₂, primary lipid molecules (DPPC/ 1,2-distearoyl-sn-glycero-3-phosphocholine (DSPC) = 0/100, 25/75, 50/50, 75/25, 100/0 by molar ratio), DSPE-PEG and cholesterols in a molar ratio of about 0.55:about 55:about 2:about 20 according to Example 2.
FIG. 3 is graph showing the temperature release profiles of doxorubicin (DX) from the liposomes prepared in Example 3 using SA-V3-NH₂: primary lipids (DSPC:DPPC = 25:75 by molar ratio), DSPE-PEG and cholesterols in a molar ratio of about 0.55:about 55:about 2:about 20.
FIG. 4 is a graph showing the temperature release profiles of DX from the liposomes prepared in Example 4 using SA-V3-NH₂, DPPC, DSPE-PEG and cholesterols in a molar ratio of about 0.55:about 55:about 2:about 20 and lysolipid thermosensitive liposomes (LTSL).
FIG. 5 is a graph comparing stability of liposomes prepared in Example 5 using SA-V3-NH₂:DPPC:DSPE-PEG and cholesterols in a molar ratio of about 0.55:about 55:about 2:about 20 and of LTSL at a temperature of 37 °C.
FIG. 6 is a graph showing DX release kinetics of liposomes prepared in Example 6 by using SA-V3-NH₂, primary lipids (DSPC/DPPC = 25/75 in a molar ratio), DSPE-PEG and cholesterols with a molar ratio of 0.55: 55: 2: 20 at a temperature of 37 °C, 40 °C, 42 °C, or 45 °C each.
FIG. 7 is a graph showing cell toxicity according to the amount of drugs contained in liposomes prepared in Example 4, which is measured in a procedure in Example 7 at a temperature of 37 °C, 42 °C, or 45 °C.
FIG. 8 is a graph showing cellular uptake of DX due to DX entrapped liposomes (DX amount: 10ug/mL) of Example 4 measured in a procedure in Example 8 according to temperature.
FIG. 9 is an illustration showing images of a transmission electron microscopy (TEM) of the liposomes prior to DX drugs entrapment according to Example 4 and the liposomes with DX drugs entrapped according to Example 4.

### DETAILED DESCRIPTION

According to an embodiment of the present invention, a liposome includes a lipid bilayer; elastin-like polypeptide (ELPs) conjugated to hydrophobic moieties; and lipid bilayer stabilizing agents, wherein the hydrophobic moieties are packed in the lipid bilayer.

The term "lipid bilayer" as used herein indicates a membrane composed of two layers of lipid molecules. The lipid layer may have a similar thickness as that of a naturally existing bilayer, for example, a cell membrane, a nuclear membrane, or a virus envelope. Examples of the thickness of the lipid bilayer may be 10 nm or less, for example, about 1 nm to about 9 nm, about 2nm to about 8 nm, about 2 nm to about 6 nm, about 2 nm to about 4 nm, or about 2.5 nm to about 3.5 nm. The lipid bilayer prevents random diffusion of ions, proteins, and other molecules. In other words, it keeps such molecules where they are needed and prevents them from diffusing into areas where they should not be. Natural lipid bilayers are usually made mostly of phospholipids. A phospholipid has a hydrophilic head and two hydrophobic tails. When phospholipids are exposed to water, they arrange themselves into a two-layered sheet (a bilayer) with all of their tails pointing toward the center of the sheet. The center of this bilayer contains almost no water and also excludes molecules like sugars or salts that dissolve in water but not in oil. Phospholipids with certain head groups can alter the surface chemistry of a bilayer. Also, lipid tails may affect membrane properties, for instance by determining the phase of the bilayer. The bilayer can adopt a solid gel phase state at lower temperatures but undergo phase transition to a fluid state at higher temperatures. The packing of lipids within the bilayer also affects its mechanical properties, including its resistance to stretching and bending. Biological membranes typically include several types of lipids other than phospholipids. A particularly important example in animal cells is cholesterol, which helps strengthen the bilayer and decrease its permeability.

A "lipid molecule" for constructing the lipid bilayer (sometimes referred to herein as a "bilayer forming lipid" or "membrane forming lipid") may be a molecule having a hydrophilic head and hydrophobic tails, for example, a phospholipid. The lipid portion of the molecule may have, for example, about 14 to 50 carbon, such as about 16 to 24 carbon atoms. Suitable phospholipids include, for instance, phosphatidyl cholines, phosphatidyl glycerols, phoaphatidyl inositols, phosphatidyl ethanolamines and combinations thereof. In some embodiments, the bilayer forming lipids include at least one phospholipid that has two acyl groups. Also, the bilayer forming lipid, e.g., phospholipid may have a phase transition temperature of about 10 °C to about 70 °C, for example, about 20 °C to about 70 °C, about 30 °C to about 70 °C, about 40 °C to about 70 °C, about 50 °C to about 70 °C, about 60 °C to about 70 °C, about 10 °C to about 60 °C, about 10 °C to about 50 °C, about 10 °C to about 40 °C, about 10 °C to about 39 °C, about 30 °C to about 60 °C, about 30 °C to about 50 °C, about 30 °C to about 40 °C, about 30 °C to about 42 °C, about 30 °C to about 40 °C,about 30 °C to about 39 °C, about 35 °C to about 60 °C, about 35 °C to about 55 °C, about 35 °C to about 50 °C, about 35 °C to about 45 °C, about 35 °C to about 42 °C, about 35 °C to about 40 °C, about 35 °C to about 39 °C, about 38 °C to about 50 °C, about 38 °C to about 45 °C, about 38 °C to about 42 °C, or about 38 °C to about 40 °C. The acyl groups of the bilayer forming lipid (e.g., phospholipid) may be saturated or unsaturated. The bilayer forming lipid may comprise a mixture of two or more different phospholipid molecules, optionally along with other bilayer forming lipids. A lipid bilayer having various phase transition temperatures may be produced due to the mixture of two or more bilayer forming lipids (e.g., two or more different phospholipid molecules).

A phospholipid molecule may have two acyl groups, for example, one selected from the group consisting of C12 saturated chain phospholipid (Tc=10 °C), a C14 saturated chain phospholipid (Tc=24 °C), a C16 saturated chain phospholipid (Tc=41 °C), a C18 saturated chain phospholipid (Tc=55 °C), a C20 saturated chain phospholipid (Tc=65 °C), a C22 saturated chain phospholipid (Tc=70 °C), and a combination thereof, wherein the Tc refers to a phase transition temperature. Similarly, other common phospholipids that may be used include phosphatidyl glycerols, phosphatidyl inositols, phosphatidyl ethanolamines, sphingomyelins and gangliosides that, as with the phosphatidylcholines, have phase transition temperatures that vary in a similar fashion dependent on their acyl chain length.

The C16 saturated chain phospholipid may be dipalmitoylphosphatidylcholine (DPPC). DPPC is a saturated chain (C16) phospholipid with a bilayer transition temperature of about 41.5 °C. An example of the C18 saturated chain phospholipid may be 1,2-distearoyl-sn-glycero-3-phosphocholine (DSPC). DSPC is a saturated chain (C18) phospholipid with a bilayer transition temperature of about 55.10 °C.

Other membrane-forming lipid materials may be used which are not phospholipids. Such lipid materials can be used instead of, or in combination with, phospholipids. Exemplary materials which may form a solid-phase membrane include bola lipids or bacterial lipids. Additionally, block copolymers including a water-soluble polymer (e.g., polyethylene glycol) and a water-insoluble polymer (e.g., polypropylene oxide and polyethylethylene) may be employed.

As used herein, the "primary lipid" in a liposome bilayer is the main lipid component of liposome bilayer material. Thus, for example, in a liposome bilayer composed of 70 mole % phospholipid and 30 mole % cholesterol, the phospholipid is the primary lipid.

A lipid bilayer may have different phase behaviors that change with temperature. At a given temperature, a lipid bilayer may exist in either a liquid or a gel (solid) phase. All lipids have a characteristic temperature at which they show transition from the gel to liquid phase. In both phases, the lipid molecules are prevented from flip-flopping the bilayer, but in liquid phase bilayers, a given lipid will exchange locations with its neighbor. This random walk exchange allows lipids to diffuse and thus wander across the surface of the membrane. Unlike liquid phase bilayer, the lipids in a gel phase bilayer are locked in place.

The phase behavior of a lipid bilayer is largely determined by the strength of the attractive forces of Van der Waals interactions between adjacent lipid molecules. Longer tailed lipids have more area over which to interact, increasing the strength of this interaction and consequently decreasing the lipid mobility. Thus, at a given temperature, a short-tailed lipid will be more fluid than an otherwise identical long-tailed lipid. Transition temperature may also be affected by the degree of unsaturation of the lipid tails. An unsaturated double bond may produce a kink in the alkane chain, disrupting the lipid packing. This disruption creates extra free space within the bilayer which allows additional flexibility in the adjacent chains.

Most natural membranes are a complex mixture of different lipid molecules. If some of the components are liquid at a given temperature while others are in the gel phase, the two phases can coexist in spatially separated regions, rather like an iceberg floating in the ocean.

As used herein, the term "phase transition temperature" or "Tc" indicates the temperature at which a material changes from a solid phase to a liquid phase (also called a melting temperature) or from a liquid phase to a solid phase. The phase transition temperature of a lipid membrane can be determined by differential scanning calorimertry (DSC), electron spin resonance(ESR) and the like. Differential scanning calorimetry may be performed with a model 4207 heatflow calorimeter from Hart Scientific. Samples may be prepared as described below, and the scan rate used may be 10°/h. Transition enthalpies may be estimated by using the peak integration software provided with the instrument. Transition entropies may be estimated from the transition enthalpies, assuming a first-order transition according to the expression ΔHₜ = Tₜ ΔSₜ, where ΔHₜ, ΔSₜ, and Tₜ, may be the transition enthalpy, entropy, and transition temperature, respectively. Samples for differential scanning calorimetry may be prepared by weighing about 3 to about 8 mg of the lipid into the DSC sample ampules and adding 0.5 ml of 10mM HEPES buffer, 1mM EDTA (pH 7.4), with or without 1 M NaCl. The ampules may be then tightly sealed with screw caps and placed in the calorimeter. The reference ampule contained an equal volume of the buffer alone. Hydration of the liquid may be achieved by heating the samples above the chain-melting phase transition temperature (to 95 °C) in the calorimeter, incubating for 20 min, then cooling to 20 °C or lower and incubating for a further 20 min. After this hydration procedure, the samples may be subjected to two heating and two cooling scans, with nearly identical results for the repeated and repeated cooling scans.

The liposome includes ELPs conjugated to hydrophobic moieties, wherein the hydrophobic moieties are packed in the lipid bilayer.

The hydrophobic moiety may be a molecule having a property of immobilizing the ELPs conjugated thereto to the lipid bilayer, for example, a hydrophobic property. The hydrophobic moiety may be the same lipid molecule composing the lipid portion of lipid bilayer, or the hydrophobic moiety may be a different type of hydrophobic moiety.

The hydrophobic moiety may be provided by a molecule only containing a hydrophobic region, or by amphipathic molecules containing both hydrophilic and hydrophobic regions. In the amphipathic molecules containing both hydrophilic and hydrophobic regions, the hydrophobic region may be arranged inwardly of the lipid bilayer, and the hydrophilic region may be arranged outwardly of the lipid bilayer and linked with ELPs. Here, "outwardly" of the lipid bilayer indicates a direction away from a center of the lipid bilayer, that is, inward of the liposome (the interior space encapsulated by the bilayer) or outward of the liposome (e.g, in, on, or protruding from the outer surface of the liposome).

The hydrophobic moiety may be lipid molecules naturally existing in biomembranes, or lipid molecules not naturally existing in biomembranes.

The lipid molecules naturally existing in biomembranes may be selected from phospholipids or their derivatives, sterols or their derivatives, sphingolipids or their derivatives, and combinations thereof. The phospholipids or their derivatives may be selected from the group consisting of phosphatidyl cholines, phosphatidyl glycerols, phosphatidyl inositols, phosphatidyl ehtanolamines and combinations thereof. The sterols or their derivatives may be cholesterols or their derivatives, or squalenes or their derivatives. The sphingolipids may be sphingomyelins or their drivatives, or gangliosides or their derivatives. The phospholipids, sterols, or sphingolipids include intermediates or precursors produced during a synthesis process in vivo. For example, the hydrophobic moiety includes phosphoglycerides, sphingosines, ceramides, or cerebrosides.

The hydrophobic moiety may be a saturated or unsaturated hydrocarbon, saturated or unsaturated acyl molecule, or saturated or unsaturated alkoxy molecule.

A conjugation of a hydrophobic moiety and an ELP may be faciliated via a non-cleavable linkage (e.g., a linkage that is not cleaved under physiological and pathological conditions, such as upon administration to an animal, mammal, or human) or by a cleavable linkage (e.g., a linkage that is cleaved upon administration to an animal, mammal, or human, perhaps only under certain conditions). An example of the cleavable linkage may be a linkage mediated by a pH cleavable linker, a heat cleavable linker, a radiation cleavable linker, or a linker that is cleaved in aqueous solution.

The hydrophobic moiety may be conjugated or bound to the ELP by way of a nitrogen atom at the N-terminus of the ELP, or a carbonyl (-C(O)-) group at the C-terminus of the ELP. Alternatively, or in addition, the hydrophobic moiety may be conjugated to the ELP by interaction with a functional group on a side chain of the ELP, such as an amino group, a carbonyl group, a hydroxyl group, a thiol group, or some combination thereof. The hydrophobic moiety may be conjugated to the ELP by an amine bond or amide bond with a nitrogen atom of the ELP, or by an amide or ester bond with the carbonyl group at the C-terminus of the ELP.

Furthermore, the ELP may be conjugated to any part of the hydrophobic moiety. For instance, if the hydrophobic moiety is branched or has multiple hydrophobic chains, the ELP can be conjugated or bound to any one or more branches or chains of the hydrophobic moiety. Furthermore, if the hydrophobic moiety is provided by an amphiphilic molecule, such as a phospholipid, the ELP can be conjugated to the hydrophilic or hydrophobic portion of the amphiphilic molecule.

The hydrophobic moiety may have 4 to 30 carbon atoms, for example, 14 to 24 carbon atoms or 16 to 24 carbon atoms. The hydrophobic moiety may be, for example, myristoyl (C14), palmitoyl (C16), stearoyl (C18), arachidonyl (C20), behenoyl (C22), or lignoceroyl (C24). The hydrophobic moiety may be packed in a lipid bilayer by a hydrophobic effect, and accordingly, the ELP conjugated to the hydrophobic moiety may be immobilized on the liposome.

As used herein the term "elastin-like polypeptides" refers to a class of amino acid polymers that undergo a conformation change dependent upon temperature and which exhibit inverse phase transitioning behavior. Inverse phase transitioning behavior indicates that the ELPs are soluble in aqueous solutions below an inverse transition temperature (Tₜ), but the ELPs are insoluble as the temperature is raised higher than Tₜ. By increasing the temperature ELPs transition from elongated chains that are highly soluble into tightly folded aggregates with greatly reduced solubility. Such inverse phase transition may be induced by ELP structures having more β-turn structures and distorted β-structures as temperature increases. In some cases, ELPs may be defined based on the phase transitioning temperature. For example, in some cases, the phase transition may occur at a temperature within a range from about 10 to about 70°C.

When ELPs are linked to the compartments of a lipid bilayer, the inverse phase transitioning behavior may destroy the lipid bilayer due to shrinkage and self-assembly of the ELPs as temperature rises from a temperature lower than Tₜ of ELP to a higher temperature. Destroying the lipid bilayer may increase the permeability of the lipid bilayer. Thus, active agents contained in a liposome including the lipid bilayer may be released with a higher permeability from the liposome. However, one or more embodiments of the present invention are not limited to any particular mechanism.

The destruction of the lipid bilayer in a liposome due to the inverse phase transitioning behavior of ELP may differ according to lipid molecules of the lipid bilayer, or the phase transition temperature of the lipid bilayer. A lipid bilayer exists in a gel phase at the phase transition temperature or below and in a liquid (crystalline) phase at the phase transition temperature or above. When the lipid bilayer exists in a gel phase, destruction of the lipid bilayer may not occur or may be limited, though a structure of ELP changes to have a β-turn structure due to the inverse phase transitioning behavior. On the other hand, when the lipid bilayer exists in a liquid phase, the destruction of the lipid bilayer may be induced as a structure of ELP changes to have a β-turn structure due to the inverse phase transitioning behavior. In other words, when the lipid bilayer exists in a liquid phase rather than in a gel phase, the inverse phase transition induces destruction of the lipid bilayer more efficiently. Therefore, a releasing temperature of active agents contained in a liposome may be controlled by adjusting the phase transition temperature of a lipid bilayer of the liposome or the inverse phase transition temperature of ELP. For example, the phase transition temperature of a lipid bilayer or a liposome including ELPs may be within a range from about 10 °C to about 70 °C, for example, about 39 °C to about 45 °C.

A liposome including ELPs according to one or more embodiments may be used for efficiently releasing active agents contained in the liposome compared to a liposome not including ELPs but only a lipid bilayer. When simply a phase transition of lipid molecules of a lipid bilayer is used, the release of active agents in a liposome is induced by dispersion of the lipid molecules. Meanwhile, when a liposome including ELPs is used, a further release of active agents may be induced by the inverse phase transition behavior of ELP, in other words, further release of active agents may be induced by a destroyed lipid bilayer due to shrinkage and assembly of ELPs. Here, the active agents may be contained in an interior space of the liposome (e.g., the space surrounded by the lipid bilayer; typically polar, hydrophilic, or water-soluble active agents), in an interior region of the lipid bilayer (e.g., the hydrophobic or non-polar region of the lipid bilayer; typically non-polar, hydrophobic, or water-insoluble active agents), on the surface of lipid bilayer or some combination thereof (e.g., multiple active agents, or active agents that are only partly hydrophilic or partly water-soluble).

The liposomes may include stabilizing agents. In the case of liposomes including ELPs, when lipid bilayer stabilizing agents employed to increase stability of a lipid bilayer are present in the lipid bilayer, the active agents may be efficiently released. The stabilizing agents may be lipids which have a phase transition temperature of the lipid bilayer or higher, preferably higher. The lipid bilayer stabilizing agents may be one selected from the group consisting of steroids or their derivatives, sphingolipids or their derivatives, and combinations thereof. The lipid bilayer stabilizing agents may be steroids with a property enabling incorporation into a lipid bilayer. The phrase "a property enabling incorporation into a lipid bilayer" used herein refers to a hydrophobic property enabling incorporation into a lipid bilayer. As used herein, the term "steroid" indicates a type of organic compound including a core of gonane or a skeleton derived therefrom that contains a specific arrangement of four cycloalkane rings that are joined to each other, in other words, three cyclohexane rings designated as rings A, B, and C from left to right, and one cyclopentane ring (the D ring). Here, "a skeleton derived therefrom" includes an unsaturated bond inserted in the gonane skeleton. The steroids may vary in terms of the functional groups attached to the four ring core and the oxidation state of the rings. For example, the steroids may include a hydrophilic functional group on the ring. For example, the steroids may have a hydroxyl group. The steroids may be sterols. The term "sterol" is a type of steroid which has the hydroxyl group at position C-3 and has a skeleton derived from cholestane. Here, the term "derived skeleton" includes an unsaturated bond inserted in the cholestane skeleton. The steroids include steroids found in plants, animals, and fungi. For example, all steroids may be made in cells either from lanosterol as in animals and fungi, or from cycloartenol as in plants. The sterols include cholesterols or their derivatives. Here, "derivative" means a derivate of cholesterol which maintains a property to be inserted in a lipid bilayer. The stabilizing agents may be one selected from the group consisting of cholesterols, sitosterols, ergosterols, stigmasterols, 4,22-stigmastadien-3-ones, stigmasterol acetates, lanosterols, cycloartenols, and combinations thereof.

When a liposome including a simple lipid bilayer not including ELPs contains the stabilizing agents, for example cholesterols, the release of active agents may be significantly reduced. Thus, in the case of a liposome including ELPs, by using lipid bilayer stabilizing agents, active agents may be efficiently released while maintaining stability of a lipid bilayer or of the liposome. In particular, in a narrow range of temperature, for example in a range of about 39 °C to about 45 °C, drugs may be efficiently released.

An ELP may be defined by its amino acid sequence. For example, a part of or an entire ELP may include one or more repeating units of VPGXG (SEQ ID NO: 1), PGXGV (SEQ ID NO: 2), GXGVP (SEQ ID NO: 3), XGVPG (SEQ ID NO: 4), GVPGX (SEQ ID NO: 5), including inverse sequences thereof, or combinations thereof, where V is valine, P is proline, G is glycine, and X is any natural or non-natural amino acid except proline. Thus, for instance, X can be Alanine (A), Arginine (R), Asparagine (N), Aspartic acid, (D), Cysteine (C), Glutamic acid (E), Glutamine (Q), Glycine(G), Histidine (H), Isoleucine (I), Leucine (L), Lysine (K), Methionine (M), Phenylalanine (F), Serine (S), Threonine (T), Tryptophan (W), Tyrosine (Y), or Valine (V); or X can be a non-natural amino acid, for instance, a synthetic amino acid having properties similar to any of the foregoing naturally occurring amino acids. In some embodiments, X is Valine, or Alanine (A). Here, X in each repeating unit may be the same or different amino acid.

The repeating units may be separated by one or more amino acids that do not remove a phase transition property of an obtained ELP, or an end portion may become the one or more amino acids or other linker moieties. A ratio of the repeating units verses the other amino acids or linker moieties may be about 0.1 to about 99.9% of the repeating units out of both the repeating units and the other amino acids. The selected repeating unit or units may be repeated twice or more, for example, about 2 to 200 times or or more. In some embodiments, the selected repeating unit or units may be repeated (individually or collectively) 4 or more times, 10 or more times, 15 or more times, 20 or more times, 50 or more times, etc.. Also, in some embodiments, the selected repeating unit or units may be repeated (individually or collectively) 200 or fewer times, for example, 150 or fewer times, 100 or fewer times, 75 or fewer times, etc.

In an embodiment of the present invention, the ELP may include blocks where any one or more of VPGXG, PGXGV, GXGVP, XGVPG, GVPGX or a combination thereof is tandemly repeated. As long as the inverse phase transition behavior is maintained, the ELP may be composed of VPGXG, PGXGV, GXGVP, XGVPG, GVPGX or combinations thereof and may include another portion in a molecule, for example a linker and blocking group. The ELP can comprise one or more repeating units that are combinations of the motifs VPGXG, PGXGV, GXGVP, XGVPG, and GVPGX. Thus, by way of non-limiting illustration, a repeating unit might have one of the following formulas: [VPGXG]ₙ, [PGXGV]ₙ, [GXGVP]ₙ, [XGVPG]ₙ, [GVPGX]ₙ, ([VPGXG] [PGXGV])ₙ, ([VPGXG][GXGVP])ₙ. ([VPGXG][XGVPG])ₙ, ([VPGXG][GVPGX])ₙ, or any other combination of two or more repeating units identified herein, wherein n is an integer of 2-200. The ELP can also comprise blocks of repeating units of different types, for instance, [VPGXG]ₙ - [PGXGV]ₘ - [GXGVP]ₚ, or any other combination of the repeating units set forth herein, wherein n, m, and p are independently selected integers of 2-200.

An N-terminus or C-terminus of the ELP may be linked with a hydrophobic moiety. Also, a hydrophobic moiety may be conjugated to an ELP by linking with a reactive group among a side chain of amino acid residue in the ELP. The reactive group may be an amino group, a hydroxyl group, a thiol group, or a carboxyl group. The other terminus not linked with a hydrophobic moiety may be blocked or unblocked. For example, when a hydrophobic moiety and an ELP are linked via the N-terminus of the ELP, a carboxyl group of the C-terminus of ELP may be blocked or unblocked. The blocking may be enabled by linking or interacting with a material that may be biocompatible, non-immunogenic, helpful in a specific delivery, or avoidable from biological degradation system. For example, the blocking may be enabled by an amide bond formed by binding a carboxyl group of a C-terminus of ELP and an amino group. The amino group may be an ammonia molecule, a primary amine, a secondary amine, or a tertiary amine. The primary, secondary, or tertiary amine may each have 1 to 10 carbon atoms, for example, 1 to 6 carbon atoms. X may be valine or alanine.

The repeating units may be each independently included in an ELP with one or more integer number of repetition. The number of repetitions may be each independently an integer of 2 to 200, 2 to 100, 2 to 80, 2 to 60, 2 to 40, 2 to 10, 2 to 12, 2 to 8, 2 to 6, 4 to 100, 8 to 80, 10 to 60, 12 to 40, 20 to 40, 4 to 10, 4 to 8, or 4 to 6.

In the liposome, a molar ratio of primary lipid molecules of the lipid bilayer: ELPs conjugated to a hydrophobic moiety may be appropriately selected according to a property of the selected lipid bilayer and a property of the ELPs conjugated to a hydrophobic moiety. For example, a molar ratio of primary lipid molecules: ELPs conjugated to a hydrophobic moiety may be about 50 to about 99.9: about 0.1 to about 50. For example, a molar ratio of primary lipid molecules (DPPC or mixtures of DPPC and DSPC): ELPs conjugated to a hydrophobic moiety (palmitoyl(VPGXG)n or stearoyl(VPGXG)n, where n is 2 to 12) may be about 50 to about 99.0: about 0.1 to about 50.

In the liposome, the lipid bilayer may include lipid bilayer stabilizing agents in the midst of the lipid bilayer to increase stability of the lipid bilayer. The stabilizing agents may be lipids having a higher phase transition temperature than a phase transition temperature of the lipid bilayer. The stabilizing agents may be sterols or glycolipids. The sterols may be cholesterols or their derivatives. The stabilizing agents, for example cholesterols, may help to strengthen the lipid bilayer and reduce its permeability. Therefore, the stabilizing agents, for example cholesterols, enable liposomes to exist stably at normal body temperature. A molar ratio of primary lipid molecules: the stabilizing agents, for example cholesterols, may be about 50 to about 99.9: about 0.1 to about 50. The ratio of the primary lipid molecules: the stabilizing agents may be about 50 to about 99.9: about 0.1 to about 50, for example about 50 to about 99.9: about 3 to about 50, about 50 to about 99.9: about 5 to about 50, about 50 to about 99.9: about 7 to about 50, about 50 to about 99.9: about 9 to about 50, about 50 to about 99.9: about 11 to about 50, about 50 to about 99.9: about 15 to about 50, about 50 to about 99.9: about 20 to about 50, about 50 to about 99.9: about 20 to about 35, about 50 to about 99.9: about 20 to about 30, about 50 to about 99.9: about 25 to about 30, about 50 to about 99.9: about 25 to about 50, about 50 to about 99.9: about 30 to about 50, about 50 to about 99.9: about 35 to about 50, about 50 to about 99.9: about 1 to about 35, about 50 to about 99.9: about 3 to about 30, about 50 to about 99.9: about 5 to about 25, about 50 to about 99.9: about 7 to about 20, or about 50 to about 99.9: about 9 to about 15.

Liposomes may not accumulate in leaky tumor tissue because of their relatively short half life in blood circulation due to their rapid uptake by macrophages of the liver and spleen (organs of the endothelial system or reticuloendothelial system (RES)). Liposome preparation may be devised to avoid rapid RES uptake and thus increase circulation times. The lipid bilayer may contain, for example, lipids derivatives derivatized with hydrophilic polymers, for example phospholipids derivatives. The hydrophilic polymers may be selected from polyethylene glycol (PEG), polylactic acid, polyglycolic acid, copolymers of polylactic acid and polyglycolic acid, polyvinyl alcohols, polyvinyl pyrrolidone, oligosaccharide and mixtures thereof. The derivatives may be phospholipids of C4-C30, for example C16-C24, conjugated with PEG. The derivatives may be DPPC-PEG or DSPE-PEG. The PEG may have a molecular weight of about 180 to about 50,000 Da.

The liposomes may be unilamellar vesicles (SUV) or multivesiclular vesicles. A diameter of the liposomes may be about 50 nm to about 500 nm, for example, about 50 nm to about 400 nm, about 50 nm to about 300 nm, about 50 nm to about 200 nm, about 100 nm to about 500 nm, about 100 nm to about 400 nm, about 100 nm to about 300 nm, or about 100 nm to about 200 nm.

In an embodiment of the present invention, the lipid bilayer may include phospholipids, ELPs conjugated to a hydrophobic moiety, phospholipid derivatives derivatized with hydrophilic polymers, and cholesterols. The phospholipids, ELPs conjugated to a hydrophobic moiety, phospholipid derivatives derivatized with hydrophilic polymers, and cholesterols in the lipid bilayer is as mentioned above.

In the embodiment, the phospholipid:ELPs with a hydrophobic moeity:phospholipid derivatives derived with hydrophilic polymers:and cholesterols may have a molar ratio of about 50 to about 99.9: about 0.1 to about 50:about 0 to about 10:about 0.1 to about 50, for example, about 50 to about 99.9:about 0.1 to about 50:about 0 to about 10:about 20 to about 50, about 50 to about 99.9:about 0.1 to about 50:about 0 to about 10:about 20 to about 30, about 50 to about 99.9:about 0.1 to about 50: 0 to about 10:about 25 to about 30, about 50 to about 99.9:about 0.1 to about 50:about 0 to about 10:about 20 to about 50, about 50 to about 99.9:about 0.1 to about 50:about 0 to about 10:about 20 to about 30, or about 50 to about 99.9:about 0.1 to about 50:about 0 to about 10:about 25 to about 30.

The phospholipid may be DPPC. The phospholipid may be a mixture of DPPC and DSPC. The phospholipid may have a molar ratio of DPPC:DSPC that is about 1:about 0 to about 0.5, for example, about 1:about 0.1 to about 0.5. The ELP conjugated to a hydrophobic moiety may include: the hydrophobic moiety having a acyl group, the ELP including (VPGXG)n or (GVPGX)m, wherein X is an amino acid except proline, and n or m is 1 or a greater integer. X may be valine or alanine. n may be 1 to 12, and m may be 1 to 12. The ELP conjugated to a hydrophobic moiety may be stearoyl-(GVPGX)2-6. A carboxyl group at the carboxyl end of the stearoyl-(GVPGX)2-6 may be blocked or not. The blocking may be blocked by an amide bond formed between a carboxyl group and an amino group (example: ammonia).

The phospholipid derivatives derivatized with hydrophilic polymers may be DPPC-PEG or DSPE-PEG. The PEG may have a molecular weight of about 180 Da to about 50,000 Da.

The liposomes according to an embodiment may have a phase transition temperature of about 10 °C to about 70 °C, for example, about 10 °C to about 60 °C, about 10 °C to about 55 °C, about 10 °C to about 45 °C, about 20 °C to about 60 °C, about 20 °C to about 55 °C, about 30 °C to about 45 °C, about 30 °C to about 45 °C, about 35 °C to about 45 °C. The phase transition temperature may be adjusted by length of a carbon chain of primary lipid molecules, number of unsaturated bonds, mixtures of lipid molecules, and combinations thereof. For example, when DSPC with a phase transition temperature higher than that of DPPC is mixed with DPPC with a lower phase transition temperature, liposomes composed of the DPPC and DSPC mixture may have a higher phase transition temperature than that of liposomes only composed of DPPC. The liposomes may be in a gel phase at room temperature.

The liposome according to an embodiment may further contain active agents. The active agents may be entrapped within the liposome interior. The active agents may be entrapped in the lipid bilayer of the liposome.

The active agents may be pharmacologically active agents or diagnostic agents. The pharmacologically active agents may be selected from the group consisting of anesthetics, antihistamines, antineoplastics, anti-ulceratives, anti-seizure agents, muscle relaxants, immunosuppressive agents, anti-infective agents, non-steroidal anti-inflammatory agents, imaging agents, nutritional agents, and combinations thereof. The active agents may be selected from the group methotrexate, doxorubicin, epirubicin, daunorubicin, vincristine, vinblastine, etoposide, ellipticine, camptothecin, paclitaxel, docetaxel, cisplatin, prednisone, methyl-prednisone, ibuprofen and combinations thereof.

According to another embodiment of the present invention, a pharmaceutical composition for delivering the active agents to a target site in a subject includes pharmaceutically acceptable carriers or diluents, and liposomes containing active agents. The liposome includes a lipid bilayer; ELPs conjugated to a hydrophobic moiety; and stabilizing agents, wherein the hydrophobic moiety may be packed in the lipid bilayer.

The pharmaceutically acceptable carrier or diluent may be well known in the art. The carrier or diluent may be selected from the group consisting of water, for example saline or sterile water, Ringer's solution, buffered saline, dextrose solution, maltodextose solution, glycerol, ethanol, and combinations thereof.

The liposomes may be dispersed in an aqueous medium. The aqueous medium may include physiological saline or PBS.

The active agents may be entrapped within the liposome interior. The active agents may be entrapped in the lipid bilayer of the liposome. The liposome may have a phase transition temperature of about 39 °C to about 45 °C. The liposome may be in a gel phase at room temperature.

The active agents may be pharmacologically active agents or diagnostic agents. The pharmacologically active agents may be selected from the group consisting of anesthetics, antihistamines, antineoplastics, anti-ulceratives, anti-seizure agents, muscle relaxants, immunosuppressive agents, anti-infective agents, non-steroidal anti-inflammatory agents, imaging agents, nutritional agents, and combinations thereof. The active agents may be selected from the group methotrexate, doxorubicin, epirubicin, daunorubicin, vincristine, vinblastine, etoposide, ellipticine, camptothecin, paclitaxel, docetaxel, cisplatin, prednisone, methyl-prednisone, ibuprofen and combinations thereof.

According to another embodiment of the present invention, the liposomes are for use in a method of delivering active agents to a target site in a subject by administering liposomes containing the active agents to a subject, wherein each liposome includes a lipid bilayer, ELPs conjugated to a hydrophobic moiety, and stabilizing agents, wherein the hydrophobic moiety is packed in the lipid bilayer; and heating the target site of a subject to release the active agents from the liposomes at the target site.

The method includes administering liposomes containing active agents to the subject. The liposomes containing the active agents have already been described above. Each liposome may have a phase transition temperature of from about 39 °C to about 45 °C.

The administration may be parenteral administration. The parenteral administration, for example, may be intravenous, intradermal, intramuscular, intracavity (abdominal cavity, joints, or eye), or direct injection. The direct injection may involve injecting directly into a diseased site such as a tumor site. The liposomes may be administered intravenously and thereby brought to the target site such as a tumor site by blood flow. The target site may have a leaky property. The phrase "leaky property" used herein refers to a property having an increased permeability of a material compared to a normal tissue or a cell. The target site may be tumor site, where the material permeability of blood vessels in tumors are increased due to the leakiness of tumor vessels.

The method includes heating the target site of the subject to release the active agent from the liposomes at the target site. The heating may be due to a clinical procedure that induces hyperthermia or may be related to an intrinsically higher temperature of an inflamed body part compared to the rest of the body. The clinical procedure that induces hyperthermia may be performed by direct heat transfer, for example, a hot liquid medium in a tub, e.g., contacting a body in water, irradiating ultrasound, e.g., high intensity ultrasound focused at a target site, applying a magnetic field, e.g., an amplified magnetic field, applying microwave and/or radio-frequency. The target site may be a region where pathological symptoms exist, for example, a tumor site (i.e., a solid tumor), or where inflammation exists. The heating may be heating to a temperature of about 38 °C to about 45 °C.

The active agents may be pharmacologically active agents or diagnostic agents. The pharmacologically active agents may be selected from the group consisting of anesthetics, antihistamines, antineoplastics, anti-ulceratives, anti-seizure agents, muscle relaxants, immunosuppressive agents, anti-infective agents, non-steroidal anti-inflammatory agents, imaging agents, nutritional agents, and combinations thereof. The active agents may be selected from the group methotrexate, doxorubicin, epirubicin, daunorubicin, vincristine, vinblastine, etoposide, ellipticine, camptothecin, paclitaxel, docetaxel, cisplatin, prednisone, methyl-prednisone, ibuprofen and combinations thereof.

The permeability of liposomes, according to an embodiment, may be adjusted by shrinking and self-assembling of ELPs conjugated to a hydrophobic moiety depending on a temperature. Therefore, the liposome may be used as a vehicle for effectively delivering an active agent to a target site of a subject.

The permeability of the liposomes containing active agents may be adjusted by a phase transition temperature of ELP conjugated to a hydrophobic moiety as well as a phase transition temperature of liposome itself. Thus, when the liposomes have a more stable composition at body temperature, for example, even at a status containing an effective amount of stabilizing molecules, such as cholesterols, for maintaining liposomes more stably at body temperature, the permeability may be efficiently adjusted by the phase transition temperature of ELP conjugated to a hydrophobic moiety.

According to another embodiment of the present invention, a method of preparing a liposome comprising combining one or more bilayer-forming lipids; one or more elastin-like polypeptides (ELPs) each conjugated to a hydrophobic moiety; and one or more lipid bilayer stabilizing agents; to provide a liposome. The liposome used in the method may comprise a lipid bilayer, and the hydrophobic moiety conjugated to the one or more ELPs is in the bilayer. The one or more bilayer-forming lipids may comprise a phospholipid.

The method may further comprise combining one or more active agents with the one or more bilayer-forming lipids, one or more ELPs each conjugated to a hydrophobic moiety, and one or more lipid bilayer stabilizing agents, or combining one or more active agents with the liposome, to provide a liposome containing the one or more active agents.

The method may comprise combining the one or more bilayer-forming lipids provided in a first solvent with the one or ELPs each conjugated to a hydrophobic moiety and the one or more lipid stabilizing agents provided in a second solvent; evaporating the combined solvents to provide a lipid layer; hydrating the lipid layer with an aqueous solvent; and filtering the hydrated lipid layer to provide a liposome. The aqueous solvent may include water or a solution in which the solvent is water, for example, physiological saline, PBS, 300 mM of citrate solution (pH 4.0), and 250 mM of ammonium sulfate solution.

The aqueous solvent may contain one or more active agents. The method may comprise combining one or more active agents with the liposome in the presence of pH gradient or ammonium sulfate gradient between the inside and outside of the liposome.

According to a pharmaceutical composition for delivering active agents containing liposomes, according to another embodiment, to a subject, the composition may be used to efficiently deliver the active agents to the subject. According to another embodiment, the compositions may be used to administer the active agents to the target sites in the body of the subject, so that the active agents may be efficiently delivered to the target sites in the body of the subject.

The present invention will now be described more fully with respect to exemplary embodiments.

### Example 1: Preparation of Liposomes and Measurement of Thermal Sensitivity

Liposomes in a form of unilamellar vesicles were prepared using stearoyl-VPGVG VPGVG VPGVG VPGVG VPGVG VPGVG-NH₂ (SEQ ID NO: 6, hereinafter referred to as "SA-V6-NH₂"), 1,2-dipalmitoyl-sn-glycero-3-phosphocholine (DPPC), [1,2-distearoyl-sn-glycero-3-phosphoethanolamine-N-[methoxy(polyethylene glycol)-2000] (ammonium salt)] (DSPE-PEG-2000), which are purchased from Peptron Co. Ltd. (Rupublic of Korea), and cholesterols in a molar ratio of 0.55:55:2:20 or 0.55:55:2:40.

In detail, SA-V6-NH₂ was dissolved in alcohol, and DPPC, DSPE-PEG and cholesterols were dissolved in chloroform. After mixing the ethanol and chloroform solution in a round-bottom flask, a lipid thin layer was formed on the interior wall of the flask by evaporating the solvent at room temperature using a rotary evaporator.

Next, the liquid thin layer was hydrated by adding physiological saline, in which 200 mM of calcein was dissolved, to the flask at room temperature. Calcein is a water-soluble fluorescence molecules.

Unilamella vesicle type liposomes were prepared by filtering the hydrated solution through a polycarbonate film with pores having a size of 100 nm. The prepared liposome solution was passed through a PD-10 (GE Healthcare) desalting column with physiological saline flow to remove unsealed calcein. As a result, liposomes with calcein entrapped in the aqueous interior were prepared. The prepared liposomes had an average diameter of about 100 nm to about 200 nm as measured by a Zeta-sizer instrument (Malvern inst.).

The in vitro stability and thermosensitivity of the prepared liposome formulations were assessed by measuring the percent release of calcein from the aqueous interior of the liposomes to the surrounding solution after 5 minutes of incubation at a temperature from about 25 °C to about 55 °C in the presence of physiological saline. The fluorescence of the calcein entrapped in the liposomes was self quenched due to its high concentration, but upon release from the liposomes and dilution into the surrounding solution, the calcein developed an intense fluorescence.

After incubation, the fluorescence intensity of the samples was measured at an excitation wavelength (λex)=485 nm and an emission wavelength (λem)=535 nm after suitable dilutions to determine the amount of calcein released from the liposomes. The relative percent fluorescence intensity due to incubation at a particular temperature was calculated by comparison with the total release of entrapped material obtained after disruption of the liposome samples by adding dimethyl sulfoxide (DMSO).

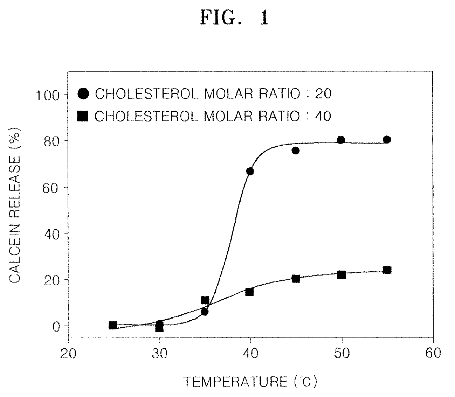

FIG. 1 is a graph showing the temperature release profiles of calcein from the liposomes prepared in Example 1 using SA-V6-NH₂, DPPC, DSPE-PEG and cholesterols in a molar ratio of about 0.55:about 55:about 2:about 20 or about 0.55:about 55:about 2:about 40. As shown in FIG. 1, the release of calcein was controlled by the amount of cholesterols at temperatures of about 35 °C to about 41 °C near the phase transition temperature of DPPC which is about 41 °C.

As shown in FIG. 1, when a molar ratio of cholesterols is 40, the release of calcein according to temperature is about 30% or less. This is because an excess amount of cholesterols contributing to stabilization of liposomes exists at a temperature of 37 °C, so the liposomes are overly stabilized, and thus liposomal destruction due to a change depending on temperature according to ELPs, for example, a shrinking phase transition, was canceled.

### Example 2:

Liposomes were prepared according to the same method used in Example 1 except 1,2-distearoyl-sn-glycero-3-phosphocholine (DSPC) was added to DPPC as a main lipid component, and primary lipid molecules were mixed and used with a molar ratio of DPPC/DSPC as 0/100, 25/75, 50/50, 75/25, 100/0. Then, thermosensitivity of the liposomes was assessed. The prepared liposomes had similar average diameter and distribution to the liposomes prepared in Example 1.

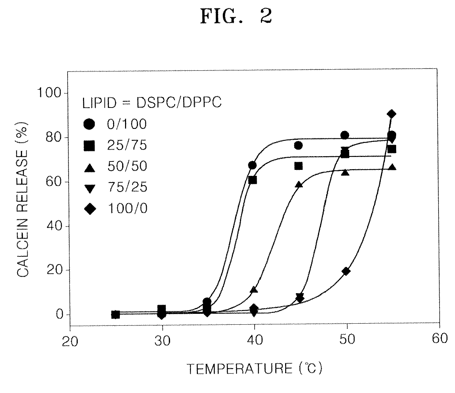

FIG. 2 is a graph showing the temperature release profiles of calcein from the liposomes prepared by using SA-V6-NH₂, primary lipid molecules (DPPC/DSPC), DSPE-PEG and cholesterols in a molar ratio of about 0.55:about 55(0/100, 25/75, 50/50, 75/25, 100/0 by molar ratio):about 2:about 20 according to Example 2. As shown in FIG. 2, as the amount of DSPC of the primary lipid molecules increased, the onset temperature of calcein release is increased. When DSPC 100 was used as the primary lipid molecules, the release of calcein started at about 40 °C and exponentially increased to about 55 °C. The maximum amount of release was 80% or more at about 55 °C. Considering the fact that a phase transition temperature of DSPC is 55 °C, such an exponential increase in the amount of release is because the phase transition temperature of all of the liposomes is considered to be about 55.1 °C. It is considered that the amount of calcein released is increased exponentially at about the phase transition temperature.

### Example 3: Preparation and Measurement of Thermosensitivity of Liposomes Containing Doxorubicin Using Ammonium Sulfate Gradient Method

DSPC and DPPC was used with a mixture ratio of 25:75 as a main lipid component, SA-V3-NH₂, DSPC+DPPC, DSPE-PEG, and cholesterols were used with a molar ratio of 0.55: 55: 2: 20, and an ammonium sulfate gradient method (J. Control. Release, 139: 73-80 (2009)) was used to prepare doxorubicin-sealed unilamellar vesicle type liposomes.

In detail, stearoyl- VPGVG VPGVG VPGVG-NH₂ (SEQ ID NO: 7, hereinafter "SA-V3-NH₂") was dissolved in ethanol, and DSPC, DPPC, DSPE-PEG and cholesterols were dissolved in chloroform. After mixing the ethanol and chloroform solution in a round-bottom flask, a lipid thin layer was formed on the interior wall of the flask by evaporating the solvent at room temperature using a rotary evaporator.

Next, the lipid thin layer was hydrated by adding 250 mM of ammonium sulfate solution to the flask at room temperature.

Unilamella vesicle type liposomes were prepared by filtering the hydrated solution through a polycarbonate film with pores having a size of 100 nm. A liposome solution formed of liposomes with 250 mM of ammonium sulfate inside and 25 mM of Tris · HCl outside by passing the prepared liposome solution through a Sephadex G-50 column filled with 25 mM of Tris · HCl. DX was added in a mass ratio of 1:0.2 to the main lipid component and incubated for an hour at a temperature of 37 °C. The prepared liposome solution was passed through a Sephadex G-50 column (GE Healthcare) filled with physiological saline to remove unsealed DX. As a result, liposomes with DX entrapped in the aqueous interior were prepared (with a sealing efficiency of 90% or higher). The prepared liposomes had an average diameter of about 170 nm as measured by a Zeta-sizer instrument (Malvern inst.).

The in vitro stability and thermosensitivity of the prepared liposome formulations was assessed by measuring the percent release of DX from the aqueous interior of the liposomes to the surrounding solution after 5 minutes of incubation at a temperature from about 25 °C to about 55 °C in the presence of physiological saline.

After incubation, the fluorescence intensity of the samples was measured at an excitation wavelength (λex)=485 nm and an emission wavelength (λem)=615 nm after suitable dilutions to determine the amount of DX released from the liposomes. The relative percent fluorescence intensity due to incubation at a particular temperature was calculated by comparison with the total release of entrapped material obtained after disruption of the liposome samples by adding 1% Triton X-100 (ethanol).

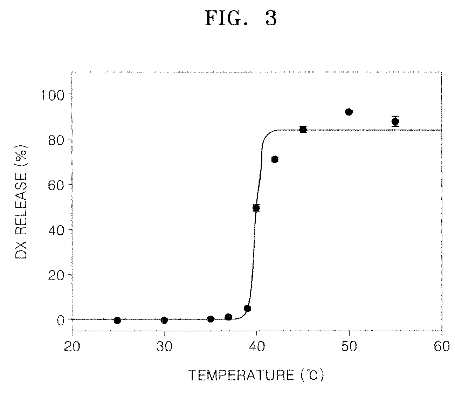

FIG. 3 is graph showing the temperature release profiles of DX from the liposomes prepared in Example 3 using SA-V3-NH₂, DSPC+DPPC, DSPE-PEG and cholesterols in a molar ratio of about 0.55:about 55:about 2:about 20. As shown in FIG. 3, the release of DX was significantly increased at temperatures of about 40 °C to about 41 °C near the phase transition temperature of DPPC, which is about 41 °C. The maximum amount of release exceeded 80%.

### Example 4: Preparation and Measurement of Thermosensitivity of Liposomes Containing Doxorubicin Using pH Gradient Method

SA-V3-NH₂, DPPC, DSPE-PEG, and cholesterols were used with a molar ratio of 0.55: 55: 2: 20, and a pH-gradient method (Biochimica et Biophysica Acta, 816: 294-302 (1985)) was used to prepare doxorubicin-sealed unilamellar vesicle type liposomes.

In detail, SA-V3-NH₂ was dissolved in ethanol, and DPPC, DSPE-PEG and cholesterols were dissolved in chloroform. After mixing the ethanol and chloroform solution in a round-bottom flask, a lipid thin layer was formed on the interior wall of the flask by evaporating the solvent at room temperature using a rotary evaporator.

Next, the lipid thin layer was hydrated by adding 300 mM of citrate solution (pH 4.0) to the flask at room temperature.

Unilamella vesicle type liposomes were prepared by filtering the hydrated solution through a polycarbonate film with pores having a size of 100 nm. A liposome solution formed of liposomes with 300 mM of citrate inside and 20 mM of HEPES (150 mM NaCl) outside was prepared by passing the prepared liposome solution through a Sephadex G-50 column filled with 20 mM HEPES (150 mM NaCl, pH 7.4). DX was added in a mass ratio of 1:0.2 to the main lipid component and incubated for 20 hours at a temperature of 37 °C. The prepared liposome solution was passed through a Sephadex G-50 column filled with physiological saline to remove unsealed DX. As a result, liposomes with DX entrapped in the aqueous interior were prepared (sealing efficiency of 90% or higher). The prepared liposomes had an average diameter of about 150 nm as measured by a Zeta-sizer instrument (Malvern inst.).

The in vitro stability and thermosensitivity of the prepared liposome formulations were assessed by measuring the percent release of DX from the aqueous interior of the liposomes to the surrounding solution after 5 minutes of incubation at a temperature from about 25 °C to about 55 °C in the presence of physiological saline.

After incubation, the fluorescence intensity of the samples was measured at an excitation wavelength (λex)=485 nm and an emission wavelength (λem)=615 nm after suitable dilutions to determine the amount of DX released from the liposomes. The relative percent fluorescence intensity due to incubation at a particular temperature was calculated by comparison with the total release of entrapped material obtained after disruption of the liposome samples by adding 1% Triton X-100 (ethanol).

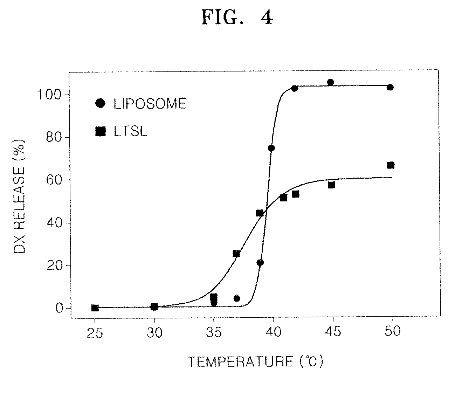

FIG. 4 is a graph showing the temperature release profiles of DX from the liposomes prepared in Example 4 using SA-V3-NH₂, DPPC, DSPE-PEG and cholesterols in a molar ratio of about 0.55:about 55:about 2:about 20. As shown in FIG. 4, the release of DX was significantly increased at temperatures about 40 °C to about 41 °C near the phase transition temperature of DPPC, which is about 41 °C. The maximum amount of release appeared to be 100%. As a control group for a comparative experiment, lysolipid thermosensitive liposomes (LTSL) prepared by the pH-gradient method using DPPC, MPPC and DSPE-PEG in a molar ratio of about 90:about 10:about 4 were used. In the case of LTSL, the drug release started at a temperature of 35 °C and continued gradual release of the drug up to 50 °C, and the maximum amount of DX release appeared to be about 60%. Comparing to the control group, the drug release profile of the liposomes containing ELPs showed a rapid release within a narrow temperature range.

### Example 5: Assessment of Stability of Liposomes

DX entrapped thermosensitive liposomes were prepared using the same procedure as used in Example 4. As a control group for a comparative experiment, LTSL prepared by the pH-gradient method using DPPC, MPPC and DSPE-PEG in a molar ratio of about 90:about 10:about 4 were used. The DX entrapped liposomes had an average diameter of about 140±10 nm.

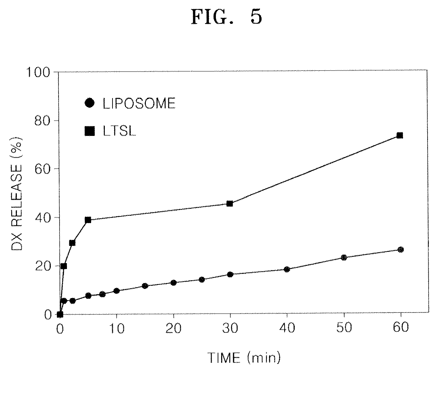

FIG. 5 is a graph showing stability of DX entrapped liposomes. FIG. 5 represents the measured amount of DX release as a function of time while the liposomes were being stored at a temperature of 37 °C. As shown in FIG. 5, the amount of drugs released from the liposomes including ELPs was significantly less than the control group. This indicates that liposomes including ELPs may be maintained stably at a temperature of 37 °C without drugs being released.

### Example 6: Drug Release Kinetics of Liposomes According to Temperature

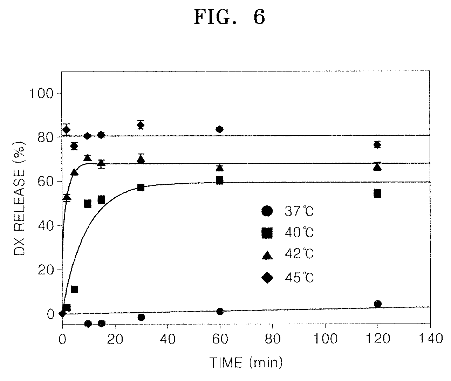

DX entrapped thermosensitive liposomes were prepared using the same procedure as used in Example 3. The release of DX was measured as a function of time at a temperature of 37 °C, 40 °C, 42 °C, or 45 °C. FIG. 6 is a graph showing DX release kinetics of liposomes prepared by using SA-V3-NH₂, primary lipid molecules (DSPC/DPPC = 25/75), DSPE-PEG, and cholesterols with a molar ratio of 0.55: 55: 2: 20 at a temperature of 37 °C, 40 °C, 42 °C, or 45 °C each. As shown in FIG. 6, the liposomes were very stable at a temperature of 37 °C where almost no leakage of drugs occurred, but with thermal stimulation at a temperature of 42 °C and 45 °C, rapid drug release was confirmed.

### Example 7: Cell Toxicity of Liposomes

DX entrapped thermosensitive liposomes were prepared using the same procedure as used in Example 4. 5.0 X 10⁴ of HeLa cells were grown in Dulbecco's modified Eagle's medium (DMEM) containing 10% (v/v) Fetal bovine serum (FBS) and 1% penicillin/streptomycin in 24-well for 24 hours. DX entrapped liposomes were treated to the grown HeLa cells according to concentration, and the cells were immediately transferred into a thermoshaker and incubated for 10 minutes at a temperature of 37 °C, 42 °C, or 45 °C. Then, the cells were grown at a temperature of 37°C for 2 hours and replaced with fresh media. Subsequently, the cells were grown at a temperature of 37 °C for 46 hours, and cell proliferation was measured by using a WST-8 (Dojindo) kit.

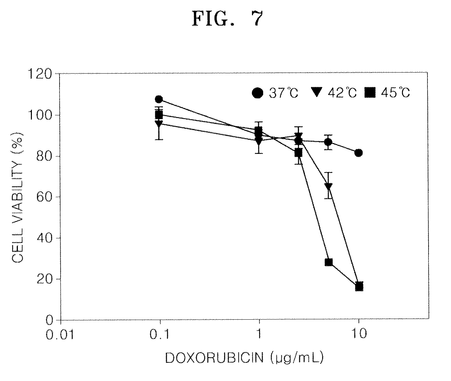

FIG. 7 is a graph showing cell toxicity according to the amount of drugs entrapped in a liposome by the method of Example 7 in which DX entrapped thermosensitive liposomes were prepared using the same procedure in Example 4. Cell viability was measured by the amount of dehydrogenase released from living cells using a WST-8 assay method. As a result of the experiment, it was confirmed that cells incubated at a temperature of 37 °C did not show toxicity, but cells incubated at a temperature of 42 °C and 45 °C showed toxicity according to concentration due to DX injected in the liposomes. Therefore, release of drugs according to temperature and inhibition of cell growth due to the released drugs were confirmed by the in vitro cell toxicity experiment.

### Example 8: Cellular Uptake of Liposomes

DX entrapped thermosensitive liposomes were prepared using the same procedure as used in Example 4. DX or DX entrapped thermosensitive liposomes were treated with HeLa cells for 2 hours. The DX concentration used was 10 ug/ml. The cells were heated at a temperature of 37 °C and 42 °C for 10 minutes, and subsequently incubated at a temperature of 37 °C for 2 hours in DMEM. The cell was rinsed with PBS and treated with 4% formaldehyde (w/v) for 30 minutes at room temperature. DAPI was used for dyeing cell nuclei. Cellular uptake was observed using a confocal laser scanning microscope (LSM 710, Carl Zeiss, USA).

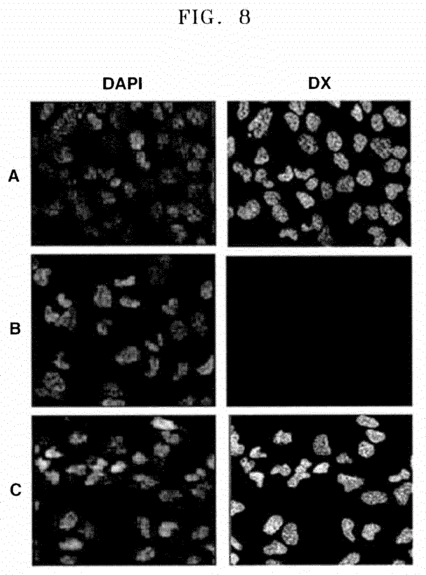

FIG. 8 is a graph showing cellular uptake of DX with the same procedure of Example 8 in which DX entrapped thermosensitive liposomes were prepared using the same procedure as used in Example 4. The DX concentration used was 10 ug/ml.

FIG. 8 shows a result of DAPI (left) or DX (right) confirmed by fluorescence after treating the DX injected liposomes in Example 4 with HeLa cells, treating the treated sample at a temperature of 37 °C (B) or 42 °C (C) for 10 minutes, incubating the treated sample in DMEM for 2 hours at a temperature of 37 °C, and rinsing the cells with PBS twice. A control group (A) shows a result of DAPI or DX confirmed by fluorescence after incubating with HeLa cells in DMEM for 2 hours at a temperature of 37 °C in presence of 10 ug/ml of DX. In FIG 8, gray area indicates cell nuclei dyed with DAPI in the left column figures, and gray area indicates cell nuclei dyed with DX in the right column figures. From this result, DX existing in cells of FIG. 8 confirms that DX is released from thermosensitive liposomes when treated at a temperature of 42 °C and then, the released DX inflows into the cells.

### Example 9: Confirmation of Liposome Morphorlogy

Morphology of liposomes prepared by the same procedure in Example 4 and thermosensitive liposomes not containing DX was confirmed using a transmission electron microscopy (cryoTEM). The liposomes were loaded on Holey carbon film-supported grids and observed. The grids were imbedded in liquid nitrogen and transferred to a cryotransfer holder (Gatan). Images were obtained by using a Tecnai F20 field emission gun TEM operating at 200kV (FEI) which was installed with a CCD camera (2k, Gatan).

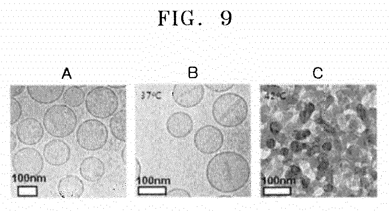

FIG. 9 is an illustration showing the images of a TEM of the liposomes prior to DX drugs entrapment according to Example 4 and the liposomes with DX drugs entrapped according to Example 4. In FIG. 9, A represents the liposomes prior to DX drugs entrapment according to Example 4 at a temperature of 37 °C, B represents the liposomes with DX drugs entrapped according to Example 4 at a temperature of 37 °C, and C represents the liposomes with DX drugs entrapped according to Example 4 at a temperature of 42 °C. As shown in FIG. 9, the liposomes prior to DX drugs entrapment according to Example 4 had a sphere shape, and maintained their morphology at a temperature of 37 °C, but the morphology was not maintained but destroyed at a temperature of 42 °C. The method used for injecting drugs was the pH-gradient method of Example 4.

## Claims

1. A liposome comprising:
a lipid bilayer; an elastin-like polypeptide (ELP) conjugated to a hydrophobic moiety, wherein the hydrophobic moiety is in the lipid bilayer; and a lipid bilayer stabilizing agent,
wherein the ELP is an amino acid polymer that is soluble in an aqueous solution below an inverse transition temperature (Tₜ) and is insoluble as the temperature is raised higher than the inverse transition temperature Tₜ.

2. The liposome of claim 1, wherein the stabilizing agent comprises a steroid; and/or is one selected from the group consisting of sterols, sphingolipids, and combinations thereof; and/or is one selected from the group consisting of cholesterols, sitosterols, ergosterols, stigmasterols, 4,22-stigmastadien-3-ones, stigmasterol acetates, lanosterols, cycloartenols, and combinations thereof.

3. The liposome of claim 1 or 2, wherein the ELP comprises one or more repeating units of VPGXG, PGXGV, GXGVP, XGVPG, GVPGX, or combinations thereof, wherein V is valine, P is proline, G is glycine, and X is any amino acid except proline, preferably wherein the repeating units are repeated 2 to 200 times.

4. The liposome of claims 1 to 3, wherein the hydrophobic moiety conjugated to the ELP comprises a hydrophobic molecule or an amphipathic molecule; and/or saturated or unsaturated hydrocarbons, saturated or unsaturated acyl molecules, or saturated or unsaturated alkoxy molecules.

5. The liposome of claim 1 to 4, wherein the lipid bilayer comprises one or more phospholipids.

6. The liposome of claim 5, wherein the one or more phospholipids comprise a phosphatidyl choline, phosphatidyl glycerol, phosphatidyl inositol, phosphatidyl ethanolamine, or a combination thereof; and/or a phospholipid comprising one or more acyl groups having 16-24 carbon atoms; and/or a phospholipid derivatized with a hydrophilic polymer, preferably wherein the hydrophilic polymer is polyethylene glycol, polylactic acid, polyglycolic acid, a copolymer of polylactic acid and polyglycolic acid, polyvinyl alcohol, polyvinyl pyrrolidone, oligosaccharide, or a mixture thereof.

7. The liposome of claims 1 to 6, wherein the lipid bilayer comprises phospholipids, phospholipids derivatized with hydrophilic polymers, and cholesterols.

8. The liposome of claims 1 to 7, wherein the liposome has a phase transition temperature of 39 °C to 45 °C, or the liposome has a diameter of 50 nm to 500 nm.

9. The liposome of claims 1 to 8, wherein the liposome further comprises one or more active agents.

10. The liposome of claim 9, wherein the one or more active agents are contained in an interior space of the liposome, in an interior region of the lipid bilayer, on the surface of the lipid bilayer, or a combination thereof; and/or the one or more active agents comprise a pharmacologically active agent, diagnostic agent, or a combination thereof, preferably the one or more active agents comprise an anesthetic, antihistamine, antineoplastic, anti-ulcerative, anti-seizure agent, muscle relaxant, immunosuppressive agent, anti-infective agent, non-steroidal anti-inflammatory agent, imaging agent, nutritional agent, or a combination thereof.

11. A pharmaceutical composition comprising:
the liposome of claim 9 and,
a pharmaceutically acceptable carrier or diluent.

12. The liposome of claim 9 for use in delivering one or more active agents to a target site in a subject.

13. A method of preparing a liposome comprising combining
one or more bilayer-forming lipids;
one or more elastin-like polypeptides (ELPs) each conjugated to a hydrophobic moiety; and one or more lipid bilayer stabilizing agents;
to provide a liposome,
wherein the ELP is an amino acid polymer that is soluble in an aqueous solution below an inverse transition temperature (Tₜ) and is insoluble as the temperature is raised higher than the inverse transition temperature Tₜ.

14. The method of claim 13, wherein the liposome comprises a lipid bilayer, and the hydrophobic moiety conjugated to the one or more ELPs is in the lipid bilayer; and/or the one or more bilayer-forming lipids comprise a phospholipid; and/or further comprising combining one or more active agents with the one or more bilayer-forming lipids, one or more ELPs each conjugated to a hydrophobic moiety, and one or more lipid bilayer stabilizing agents, or combining one or more active agents with the liposome, to provide a liposome containing the one or more active agents.

15. The method of claim 13 or 14, wherein the method comprises combining the one or more bilayer-forming lipids provided in a first solvent with the one or more ELPs each conjugated to a hydrophobic moiety and the one or more lipid stabilizing agents provided in a second solvent;
evaporating the combined solvents to provide a lipid layer;
hydrating the lipid layer with an aqueous solvent;
and filtering the hydrated lipid layer to provide a liposome;
preferably wherein the aqueous solvent contains one or more active agents.

## Patentansprüche

1. Liposom umfassend:
eine Lipiddoppelschicht; ein Elastin-ähnliches Polypeptid (ELP), das mit einem hydrophoben Rest konjugiert ist, wobei der hydrophobe Rest in der Lipiddoppelschicht ist; und
ein die Lipiddoppelschicht stabilisierendes Mittel, wobei das ELP ein Aminosäurepolymer ist, welches in einer wässrigen Lösung unterhalb einer inversen Übergangstemperatur (Tₜ) löslich ist und unlöslich ist, wenn die Temperatur über die inverse Übergangstemperatur Tₜ angehoben wird.

2. Liposom gemäß Anspruch 1, wobei das stabilisierende Mittel ein Steroid umfasst; und/oder eines ausgewählt aus der Gruppe bestehend aus Sterinen, Sphingolipiden und Kombinationen daraus ist; und/oder eines ausgewählt aus der Gruppe bestehend aus Cholesterinen, Sitosterinen, Ergosterinen, Stigmasterinen, 4,22-Stigmastadien-3-onen, Stigmasterinacetaten, Lanosterinen, Cycloartenolen und Kombinationen daraus ist.

3. Liposom gemäß Anspruch 1 oder 2, wobei das ELP eine oder mehrere sich wiederholende Einheiten aus VPGXG, PGXGV, GXGVP, XGVPG, GVPGX oder Kombinationen daraus umfasst, wobei V Valin ist, P Prolin ist, G Glycin ist und X jede Aminosäure außer Prolin ist, bevorzugt wobei die sich wiederholenden Einheiten sich 2 bis 200 Mal wiederholen.

4. Liposom gemäß den Ansprüchen 1 bis 3, wobei der hydrophobe Rest, der mit dem ELP konjugiert ist, ein hydrophobes Molekül oder ein amphipatisches Molekül umfasst; und/oder gesättigte oder ungesättigte Kohlenwasserstoffe, gesättigte oder ungesättigte Acylmoleküle oder gesättigte oder ungesättigte Alkoxymoleküle.

5. Liposom gemäß der Ansprüche 1 bis 4, wobei die Lipiddoppelschicht ein oder mehrere Phospholipide umfasst.

6. Liposom gemäß Anspruch 5, wobei das eine oder mehrere Phosphorlipid ein Phosphatidylcholin, Phosphatidylglycerin, Phosphatidylinosit Phosphatidylethanolamin oder eine Kombination daraus umfasst; und/oder ein Phospholipid umfassend ein oder mehrere Acylgruppen mit 16 bis 24 Kohlenstoffatomen; und/oder ein Phospholipid, das mit einem hydrophilen Polymer derivatisiert ist, bevorzugt worin das hydrophile Polymer Polyethylenglycol, Polymilchsäure, Polyglycolsäure, ein Copolymer aus Polymilchsäure und Polyglycolsäure, Polyvinylalkohol, Polyvinylpyrrolidon, Oligosaccharide oder eine Mischung daraus ist.

7. Liposom gemäß der Ansprüche 1 bis 6, wobei die Lipiddoppelschicht Phospholipide, Phospholipide derivatisiert mit hydrophilem Polymeren und Cholesterine umfasst.

8. Liposom gemäß der Ansprüche 1 bis 7, wobei das Liposom eine Phasenübergangstemperatur von 39° bis 45° C aufweist oder das Liposom einen Durchmesser von 50 bis 500 nm aufweist.

9. Liposom gemäß der Ansprüche 1 bis 8, wobei das Liposom weiterhin einen oder mehrere Wirkstoffe umfasst.

10. Liposom gemäß Anspruch 9, wobei der eine oder die mehrere Wirkstoffe in einem Innenraum des Liposoms, in einer inneren Region der Lipiddoppelschicht, auf der Oberfläche der Lipiddoppelschicht oder einer Kombination daraus enthalten sind; und/oder der eine oder die mehrere Wirkstoffe ein pharmkologisch wirksames Mittel, ein diagnostisches Mittel oder eine Kombination daraus umfasst, bevorzugt umfasst der eine oder die mehrere Wirkstoffe ein Anästhetikum, Antihistamin, antineoplastisches Mittel, ulkusprotektives Mittel, Antikonvulsivum, muskelentspannendes Mittel, Immunsuppressivum, Antiinvektivum, nicht-steroidales Antirheumatikum, bildgebendes Mittel, Nahrungsmittel oder eine Kombination daraus.

11. Pharmazeutische Zusammensetzung umfassend:
das Liposom gemäß Anspruch 9 und
einen pharmazeutisch verträglichen Träger oder Verdünner.

12. Liposom gemäß Anspruch 9, zur Verwendung in der Freisetzung von ein oder mehreren Wirkstoffen an der Zielstelle in einem Subjekt.

13. Verfahren zur Herstellung eines Liposoms umfassend das Zusammenbringen von einem oder mehreren Doppelschicht-bildenden Lipiden;
einem oder mehreren Elastin-ähnlichen Polypeptiden (ELPs) jeweils mit einen hydrophoben Rest konjugiert; und
einem oder mehrerer Lipiddoppelschicht-stabilisierenden Mittel;
um ein Liposom bereitzustellen, in welchem das ELP ein Aminosäurepolymer ist, welches in einer wässrigen Lösung unterhalb einer inversen Übergangstemperatur (Tₜ) löslich ist und unlöslich ist, sowie die Temperatur über die inverse Übergangstemperatur Tₜ angehoben wird.

14. Verfahren gemäß Anspruch 13, wobei das Liposom eine Lipiddoppelschicht umfasst und der hydrophobe Rest, welcher mit einem oder mehreren ELPs konjugiert ist, sich in der Lipiddoppelschicht befindet; und/oder die eine oder mehrere doppelschichtbildenden Lipide ein Phospholipid umfassen; und/oder weiterhin umfassend das Zusammenbringen eines oder mehrerer Wirkstoffe mit dem einen oder mehreren doppelschichtformenden Lipiden, einem oder mehreren ELPs, die jeweils mit einen hydrophoben Rest konjugiert sind, und einem oder mehreren doppelschichtstabilisierenden Mittel, oder das Zusammenbringen eines oder mehrerer Wirkstoffe mit dem Liposom, um ein Liposom enthaltend den einen oder mehrere Wirkstoffe bereitzustellen.

15. Verfahren gemäß Anspruch 13 oder 14, wobei das Verfahren das Zusammenbringen des einen oder der mehreren doppelschichtbildenden Lipide in einem ersten Lösungsmittel mit dem einen oder den mehreren ELPs, die jeweils mit einem hydrophoben Rest konjugiert sind, und dem einen oder den mehreren lipidstabilisierenden Mittel in einem zweiten Lösungsmittel umfasst;
Verdampfen der vereinigten Lösungsmittel, um eine Lipidschicht bereitzustellen;
Hydrieren der Lipidschicht mit einem wässrigen Lösungsmittel;
und Filtern der hydratisierten Lipidschicht, um ein Liposom bereitzustellen;
bevorzugt wobei das wässrige Lösungsmittel ein oder mehrere Wirkstoffe enthält.

## Revendications

1. Liposome comprenant:
une double couche lipidique ; un polypeptide du type élastine (ELP) conjugué à un fragment hydrophobe, le fragment hydrophobe étant dans la double couche lipidique ; et un agent stabilisant les doubles couches lipidiques, l'ELP étant un polymère d'acides aminés qui est soluble dans une solution aqueuse en-dessous d'une température de transition inverse (Tₜ) et insoluble à mesure que la température est élevée au-delà de la température de transition inverse Tₜ.

2. Liposome selon la revendication 1, l'agent stabilisant comprenant un stéroïde ; et/ou étant choisi dans le groupe constitué de stérols, sphingolipides et combinaisons de ceux-ci ; et/ou étant choisi dans le groupe constitué de cholestérols, sitostérols, ergostérols, stigmastérols, 4,22-stigmastadién-3-ones, acétates de stigmastérol, lanostérols, cycloarténols et combinaisons de ceux-ci.

3. Liposome selon la revendication 1 ou 2, l'ELP comprenant un ou plusieurs motifs répétés parmi VPGXG, PGXGV, GXGVP, XGVPG, GVPGX ou leurs combinaisons, où V est la valine, P est la proline, G est la glycine et X est un quelconque acide aminé à l'exception de la proline, les motifs répétés étant de préférence répétés de 2 à 200 fois.

4. Liposome selon les revendications 1 à 3, le fragment hydrophobe conjugué à l'ELP comprenant une molécule hydrophobe ou une molécule amphipatique ; et/ou des hydrocarbures saturés ou insaturés, des molécules acyle saturées ou insaturées, ou des molécules alcoxy saturées ou insaturées.

5. Liposome selon les revendications 1 à 4, la double couche bilipidique comprenant un ou plusieurs phospholipides.

6. Liposome selon la revendication 5, le ou les phospholipides comprenant un phosphatidylcholine, phosphatidylglycérol, phosphatidylinositol, phosphatidyléthanolamine, ou une combinaison de ceux-ci ; et/ou un phospholipide comprenant un ou plusieurs groupes acyle ayant de 16 à 24 atomes de carbone ; et/ou un phospholipide soumis à une dérivatisation par un polymère hydrophile, le polymère hydrophile étant de préférence un(e) polyéthylène glycol, acide polylactique, acide polyglycolique, copolymère d'acide polylactique et d'acide polyglycolique, alcool polyvinylique, pyrrolidone polyvinylique, oligosaccharide, ou mélange de ceux-ci.

7. Liposome selon les revendications 1 à 6, la double couche lipidique comprenant des phospholipides, des phospholipides soumis à une dérivatisation par des polymères hydrophiles et des cholestérols.

8. Liposome selon les revendications 1 à 7, le liposome ayant une température de transition de phase de 39 °C à 45 °C, ou le liposome ayant un diamètre de 50 nm à 500 nm.

9. Liposome selon les revendications 1 à 8, le liposome comprenant, en outre, un ou plusieurs agents actifs.

10. Liposome selon la revendication 9, le ou les agents actifs étant contenus dans un espace intérieur du liposome, dans une région intérieure de la double couche lipidique, sur la surface de la double couche lipidique, ou une combinaison de ceux-ci ; et/ou le ou les agents actifs comprenant un principe pharmaceutiquement actif, un agent de diagnostic, ou une combinaison de ceux-ci, le ou les agents actifs comprenant de préférence un agent anesthésique, antihistaminique, antinéoplasique, anti-ulcéreux, anti-épileptique, un myorelaxant, un agent immunosuppresseur, un agent anti-infectieux, un agent anti-inflammatoire non stéroïdien, un agent d'imagerie, un agent nutritionnel, ou une combinaisons de ceux-ci.

11. Composition pharmaceutique comprenant :
le liposome selon la revendication 9, et
un véhicule ou un diluant acceptable sur le plan pharmaceutique.

12. Liposome selon la revendication 9, destiné à être utilisé pour délivrer un ou plusieurs agents actifs au niveau d'un site cible chez un sujet.

13. Procédé de préparation d'un liposome consistant à combiner
un ou plusieurs lipides aptes à former des doubles couches ;
un ou plusieurs polypeptides du type élastine (ELP), chacun conjugué à un fragment hydrophobe ; et
un ou plusieurs agents stabilisant les doubles couches lipidiques ;
de façon à obtenir un liposome,
l'ELP étant un polymère d'acides aminés qui est soluble dans une solution aqueuse en-dessous d'une température de transition inverse (Tₜ) et insoluble à mesure que la température est élevée au-delà de la température de transition inverse Tₜ.

14. Procédé selon la revendication 13, le liposome comprenant une double couche lipidique, et le fragment conjugué à ou aux ELP se trouvant dans la double couche lipidique ; et/ou le ou les lipides aptes à former des doubles couches comprenant un phospholipide ; et/ou consistant, en outre, à combiner un ou plusieurs agents actifs avec le ou les lipides aptes à former des doubles couches, un ou plusieurs ELP conjugués chacun à un fragment hydrophobe, et un ou plusieurs agents stabilisant les doubles couches lipidiques, ou à combiner un ou plusieurs agents actifs avec le liposome, de façon à obtenir un liposome contenant le ou les agents actifs.

15. Procédé selon la revendication 13 ou 14, le procédé consistant à combiner le ou les lipides aptes à former des doubles couches lipidiques fournis dans un premier solvant avec le ou les ELP conjugués chacun à un fragment hydrophobe et avec le ou les agents stabilisant les lipides fournis dans un second solvant ;
évaporer les solvants combinés pour fournir un couche lipidique ;
hydrater la couche lipidique avec un solvant aqueux ;
et filtrer la couche lipidique hydratée pour obtenir un liposome ;
le solvant aqueux comprenant de préférence un ou plusieurs agents actifs.


<110> Samsung Electronics Co., Ltd.
<120> Liposome including elastin-like polypeptides and use thereof
<130> EP84139JH184pau
<140> not yet assigned
   <141> 2011-10-19
<150> KR10-2011-0107055
   <151> 2011-10-19
<160> 7
<170> KopatentIn 2.0
<210> 1
   <211> 5
   <212> PRT
   <213> Artificial Sequence
<220>
   <223> elastin-like polypeptide unit sequence
<220>
   <221> VARIANT
   <222> (4)
   <223> Xaa denotes amino acid other than proline
<400> 1
   Val Pro Gly Xaa Gly
    1               5
<210> 2
   <211> 5
   <212> PRT
   <213> Artificial Sequence
<220>
   <223> elastin-like polypeptide unit sequence
<220>
   <221> VARIANT
   <222> (3)
   <223> Xaa denotes amino acid other than proline
<400> 2
   Pro Gly Xaa Gly Val
    1               5
<210> 3
   <211> 5
   <212> PRT
   <213> Artificial Sequence
<220>
   <223> elastin-like polypeptide unit sequence
<220>
   <221> VARIANT
   <222> (2)
   <223> Xaa denotes amino acid other than proline
<400> 3
   Gly Xaa Gly Val Pro
    1               5
<210> 4
   <211> 5
   <212> PRT
   <213> Artificial Sequence
<220>
   <223> elastin-like polypeptide unit sequence
<220>
   <221> VARIANT
   <222> (1)
   <223> Xaa denotes amino acid other than proline
<400> 4
   Xaa Gly Val Pro Gly
   1               5
<210> 5
   <211> 5
   <212> PRT
   <213> Artificial Sequence
<220>
   <223> elastin-like polypeptide unit sequence
<220>
   <221> VARIANT
   <222> (5)
   <223> Xaa denotes amino acid other than proline
<400> 5
   Gly Val Pro Gly Xaa
   1               5
<210> 6
   <211> 30
   <212> PRT
   <213> Artificial Sequence
<220>
   <223> elastin-like polypeptide unit sequence modified with stearoylation and amidation
<220>
   <221> VARIANT
   <222> (1)
   <223> Amino terminal nitrogen is stearoylated
<220>
   <221> VARIANT
   <222> (30)
   <223> Carboxy terminal carboxy group is amidated with -NH2
<400> 6
   Val Pro Gly Val Gly Val Pro Gly Val Gly Val Pro Gly Val Gly Val
   1               5                   10                  15
   Pro Gly Val Gly Val Pro Gly Val Gly Val Pro Gly Val Gly
   20 25 30
<210> 7
   <211> 15
   <212> PRT
   <213> Artificial Sequence
<220>
   <223> elastin-like polypeptide unit sequence modified with stearoylation at amino terminal and amidation at carboxy terminal
<220>
   <221> VARIANT
   <222> (1)
   <223> Amino terminal nitrogen is stearoylated
<220>
   <221> VARIANT
   <222> (15)
   <223> Carboxy terminal carboxyl group is amidated with -NH2
<400> 7
   Val Pro Gly Val Gly Val Pro Gly Val Gly Val Pro Gly Val Gly
   1               5                   10                  15